# EUROPÄISCHE PATENTSCHRIFT

(11) **EP 3 991 758 B1**
(45) Veröffentlichungstag und Bekanntmachung des Hinweises auf die Patenterteilung: **04.12.2024**
(21) Anmeldenummer: 21200832.0
(22) Anmeldetag: 04.10.2021
(51) Int. Cl.: A61L 9/20

(54) **VORRICHTUNG ZUR REINIGUNG VON RAUMLUFT**
DEVICE FOR PURIFYING AIR IN A ROOM
DISPOSITIF DE NETTOYAGE DE L'AIR AMBIANT

(30) Priorität: 06.10.2020 DE 102020126096
(43) Veröffentlichungstag der Anmeldung: 04.05.2022
(73) Patentinhaber: youvee GmbH, 76137 Karlsruhe (DE)
(72) Erfinder: Selig, Martin, 76137 Karlsruhe (DE)
(74) Vertreter: Christ, Niko

(56) Entgegenhaltungen:
- WO-A1-2012/068569
- DE-U1- 202020 103 935
- KR-A- 20200 041 014
- KR-B1- 101 959 671
- KR-B1- 102 031 772
- US-A1- 2014 001 374
- US-A1- 2018 347 574
- US-A9- 2018 050 123

## Beschreibung

Die vorliegende Erfindung betrifft eine Vorrichtung zur Reinigung von Raumluft, umfassend ein aufrecht stehendes, rohrförmiges und aus einem opaken Material hergestelltes Luftleitelement mit einem Lufteinlass und einem Luftauslass, sowie wenigstens einem zwischen diesen angeordneten, eine in dem Luftleitelement abwärts gerichtete Strömung bewirkenden Strömungsantrieb und einer dem Luftleitelement inwendig zugeordneten Beleuchtungsvorrichtung, welche wenigstens ein UV-C-Leuchtmittel umfasst.

Eine solche Vorrichtung ist bereits aus der US 2018/0050123 A9 vorbekannt. Hinsichtlich weiteren Standes der Technik ist auf die KR 10-2020-0041014 A, die KR 10-1959671 B1, die KR 10-2031772 B1, die WO 2012/068569 A1, die US 2018/0347574 A1 und die DE 20 2020 103 935 U1 zu verweisen.

Eine ähnliche Vorrichtung ist bereits aus der US 8,900,519 B2 vorbekannt. Hierbei wird ein Luftstrom durch einen röhrenförmigen Körper geleitet, in welchem an den Wänden zahlreiche Einzel-LEDs vorgesehen sind. Diese erfordern, sowohl aus konstruktiven Überlegungen heraus und um ein Austreten des UV-C-Lichts zu vermeiden, jedoch eine vergleichsweise komplexe Bauweise, die zu einem kostenintensiven Produkt führt.

Weiter ist auf die US 2009/0041632 A1, die DE 10 2020 120 046 A1 und die US 2014/0001374 A1 zu verweisen.

Derartige Vorrichtungen sind bereits aus dem Stand der Technik bekannt und dienen im Allgemeinen zum Umwälzen der Luft, insbesondere dazu, um kühle Luft aus den Bodenschichten in höhere Bereiche anzuheben. So ist in der US 3,827,342 A eine Vorrichtung beschrieben, welche Luft bodenseitig ansaugt und mithilfe eines Strömungsantriebs in einem rohrförmigen Luftleitelement nach oben fördert. Da aufgrund der Konvektion kalte Luft nach unten sinkt, während warme Luft nach oben steigt, kann das Raumklima durch eine Umwälzung der Luftschichten im Raum verbessert werden und gerade an warmen Tagen eine Kühlung durchgeführt werden. Hierbei erfolgt in dem Luftleitelement auch eine Luftfilterung mithilfe eines Filterelements, so dass die oben ausgebrachte Luft auch entsprechend gereinigt wird.

Eine ähnliche Lösung ist aus der DE 34 00 487 A1 vorbekannt, wo ebenfalls ein Filter in einem aufrecht stehenden Luftleitelement vorgesehen ist. Auch hier erfolgt eine Umwälzung von oben nach unten.

Allerdings ergibt sich hierdurch das Problem, dass eine Durchmischung der Luft auch eine Verteilung von in der Luft vorhandenen Viren und Bakterien bewirkt. Im Zusammenhang mit dem Coronavirus ist bekannt, dass sich solche Viren nicht ohne Weiteres aus der Luft filtern lassen, sich also mit einer solchen Lösung vermehrt im Raum verteilen würde. Gerade im kritischen Kopfbereich sorgt eine solche Lösung für Verwirbelungen im Raum, welche zu einer Verteilung der mit Viren beladenen Aerosole führt.

Gerade das Coronavirus kann unter anderem über eine Tröpfcheninfektion übertragen werden, wobei die geltenden Abstandsregeln von 1,5 Metern in geschlossenen Räumen nicht ausreichend zu sein versprechen. Die Viren befinden sich in mikroskopisch kleinen Tröpfchen, die über die Atemluft ausgestoßen werden. Sie werden permanent von infizierten Personen abgegeben und schweben zunächst in der Luft, in der sie langsam absinken. Allerdings sorgen in umgekehrter Richtung Wärmequellen wie Computer, Projektoren oder sonstige Bürogeräte, Kaffeemaschinen und dergleichen für eine Luftbewegung im Raum, welche die virenbeladenen Aerosole auch wieder aufsteigen lässt und im Raum verteilt. Da aufsteigende Luft in einem geschlossenen Raum an anderer Stelle für wieder absinkende Luft sorgt, durchmischt sich die Luft im Raum fortwährend. Die eingangs genannten Geräte verstärken diesen Effekt zusätzlich.

Vor diesem Hintergrund liegt der vorliegenden Erfindung die Aufgabe zu Grunde, eine Vorrichtung zur Reinigung von Raumluft zu schaffen, welche sich auch für die Reinigung der Raumluft von Viren eignet und die Erzeugung von zusätzlichen Verwirbelungen im Raum möglichst vermeidet.

Dies gelingt durch eine Vorrichtung zur Reinigung von Raumluft mit den Merkmalen des unabhängigen Anspruchs 1. Sinnvolle Ausgestaltungen einer solchen Vorrichtung können den sich anschließenden abhängigen Ansprüchen entnommen werden.

Erfindungsgemäß ist es vorgesehen, dass eine solche Vorrichtung ein senkrecht stehendes Luftleitelement aufweist, welches einen oben liegenden Lufteinlass mit einem unten liegenden Luftauslass verbindet. Der Luftauslass wird hierbei bevorzugt direkt in Bodennähe angeordnet, so dass aus dem Luftleitelement austretende Luft keine Verwirbelungen erzeugt, sondern mehr oder weniger parallel zum Boden austreten kann. Hierdurch wird eine tiefe Luftschicht erzeugt, die sich am Boden ausbreitet und die vorhandenen Luftschichten lediglich langsam nach oben drückt. Um darüber hinaus zu vermeiden, dass eine Verteilung von Viren erfolgt, sieht die erfindungsgemäße Vorrichtung ein Luftreinigungselement in Form einer Beleuchtungsvorrichtung vor, welches aus wenigstens einer, bevorzugt aber aus mehreren UV-C-Leuchtmitteln besteht. Durch das Luftleitelement im oberen Bereich, vorzugsweise im Deckenbereich, angesaugte und aufgrund eines in dem Luftleitelement oder unmittelbar an dieses angrenzend angeordneten Strömungsantriebs im Luftleitelement abwärts gezogene Luft wird auf dem Weg zum bodenseitigen Luftauslass eine Strecke passieren, auf der sie mit der UV-C-Strahlung der UV-C-Leuchtmittel beaufschlagt wird. Die UV-C-Strahlung der Beleuchtungsvorrichtung ist sehr energiereich und bewirkt eine Zerstörung der DNS der Viren und Bakterien in Sekundenbruchteilen. Ein Einsatz von UV-C-Strahlung zur Desinfektion etwa in Operationssälen ist bereits bekannt.

Daher sieht die Erfindung auch vor, dass das Luftleitelement, welches insbesondere rohrförmig ausgebildet sein kann, aus einem opaken Material hergestellt ist, so dass eine Strahlung der UV-C-Leuchtmittel nicht nach außen dringen kann, insbesondere nicht auf Personen gerichtet wird. Auf diese Weise gelingt eine gleichmäßige Umwälzung und Reinigung der Luftmassen im Raum, wobei die Luft bodennah wieder abgegeben wird, so dass eine Verwirbelung hierbei weitestgehend vermieden wird.

Als UV-C-Leuchtmittel können prinzipiell alle bekannten Leuchtmittel eingesetzt werden, welche die benötigte UV-C-Strahlung emittieren können. Insbesondere eignen sich Leuchtdiodenanordnungen, welche um eine Zentralachse herum angeordnet sind und in alle Richtungen abstrahlen, so dass eine Beaufschlagung der gesamten durch das Luftleitelement geführten Luft erfolgt. Allerdings können gerade Leuchtdioden unterdessen in verschiedensten Formen hergestellt werden und sind klein und kostengünstig herzustellen, so dass eine nahezu beliebige Anordnung in dem Luftleitelement vorgesehen werden kann.

Auch die Verwendung von UV-C-Leuchtröhren ist jedoch möglich, wobei hier die Form weitgehend vorgegeben ist. Jedoch können diese insbesondere über lange Zeiträume hinweg günstig betrieben werden.

Bevorzugtermaßen handelt es sich bei dem Luftleitelement um ein rohrförmiges Element, welches bei einem bodennahen Luftauslass sich bis in oder über Kopfhöhe hinaus nach oben erstreckt. Vorzugsweise befindet sich der oben liegende Lufteinlass in Deckennähe. Ihm kann ein Staubfilter, vorzugsweise ein nach innen gerichteter, kegelförmiger Grobstaubfilter, zugeordnet sein, welcher Verschmutzungen im Inneren des Luftleitelements vermeidet. Derartige Verschmutzungen bewirken eine Verschlechterung der Abstrahlung der UV-C-Leuchtmittel und damit deren Einwirkung auf die Viren und Bakterien in der an den UV-C-Leuchtmitteln vorbeigeführten Luft. Insbesondere in diesem Fall müssen zudem separate Maßnahmen zur Abschirmung der UV-C-Leuchtmittel nicht notwendigerweise getroffen werden, da die Strahlung durch das Filter bereits abgefangen wird und aufgrund einer solchen Höhe des Luftleitelements mit Personen nicht in Kontakt kommt.

Um die Sicherheit zu erhöhen und um solche Luftleitelemente einsetzen zu können, welche nicht bis in Kopfhöhe reichen, ist eine Lamellenanordnung, eine Labyrinthleitung oder eine Wabenanordnung im Bereich des Lufteinlasses und/oder des Luftauslasses vorgesehen, wobei sich die UV-C-Leuchtmittel aus der Sicht des Betrachters jeweils hinter der Lamellenanordnung, der Labyrinthleitung oder der Wabenanordnung befindet, bei der beidseitigen Anordnung von Lamellenanordnungen zwischen diesen. Bei einer solchen Lamellenanordnung handelt es sich um eine oder mehrere senkrecht oder geneigt angeordnete Sichtschutzlamellen, welche dafür sorgen, dass die Strahlung der UV-C-Leuchtmittel gegen nicht aus dem Luftleitelement austreten kann. Bevorzugtermaßen kann eine solche Lamellenanordnung aus einem absorbierenden, reflexionsfreien oder reflexionsarmen Material hergestellt oder mit diesem beschichtet sein. Dies gilt auch für die genannte Wabenanordnung, welche mit besonderem Vorteil auch als Filter aus Vlies, einem Gewirk wie etwa einem Filz oder einem Textilmaterial ausgestaltet sein kann. Die Waben einer solchen Wabenanordnung können hierbei lang im Verhältnis zu ihrem Durchmesser sein, insbesondere kann die Länge wenigstens zehnmal so groß sein wie der Durchmesser. Hierdurch wird die Lichtdurchlässigkeit der Wabenanordnung verringert, da aufgrund der Länge der Waben nur noch wenige parallele Strahlenbündel durch die Wabenanordnung hindurchgelangen.

Den UV-C-Leuchtmitteln kann ferner zumindest ein zusätzlicher UV-C-Reflektor zugeordnet sein, welcher die von dem wenigstens einen UV-C-Leuchtmittel emittierte Strahlung reflektiert und erneut in den von der Luft durchströmten Bereich zurückwirft. Dies verstärkt die Wirkung der UV-C-Leuchtmittel nochmals deutlich, so dass die Wahrscheinlichkeit, mit welcher Viren und Bakterien das Luftleitmittel passieren können, weiter erheblich reduziert wird.

Ein solcher UV-C-Reflektor kann aus einem zylindrisch geformten Aluminiumblech hergestellt sein, welches zudem mit einer Beschichtung, vorzugsweise aus hochreinem Aluminium, versehen ist. Ebenso kann die Beschichtung auch direkt auf die Innenseite des Luftleitelements, insbesondere im Bereich der UV-C-Leuchtmittel, aufgebracht sein. Bevorzugtermaßen ist der UV-C-Reflektor so ausgestaltet, dass der gesamte Luftstrom durch den UV-C-Reflektor hindurchgeleitet wird und kein Anteil des Luftstroms um den UV-C-Reflektor herum geleitet wird.

Auch bei endständig offenen Luftleitelementen kann ein Sicherheitsaspekt hinzugefügt werden, wenn der Spannungsversorgung der UV-C-Leuchtmittel ein Neigungsschalter zugeordnet ist. Dieser detektiert eine Neigung des Luftleitelements und trennt die Spannungsversorgung der UV-C-Leuchtmittel bei Überschreitung einer kritischen Neigung, vorzugsweise bei einer Überschreitung von 5° gegenüber der Senkrechten. Wird also die Vorrichtung geneigt oder gar gekippt, so werden die Leuchtmittel durch den Neigungsschalter selbsttätig abgeschaltet.

Dem Luftleitelement kann zudem ein Standfuß zugeordnet sein, welcher im Wesentlichen kegelstumpfförmig oder kegelförmig gebildet ist. Allerdings weist der Kegelmantel konkave Mantelflächen auf, so dass von dem Strömungsantrieb abwärts geförderte Luft an der konkaven Mantelfläche umgelenkt und an den Boden gedrückt wird, auf dem der Standfuß steht.

Die vorstehend beschriebene Erfindung wird im Folgenden anhand eines Ausführungsbeispiels näher erläutert.

Es zeigen
- Figur 1: eine nicht erfindungsgemäße Vorrichtung in seitlicher Querschnittsdarstellung, sowie
- Figur 2: eine erfindungsgemäße Vorrichtung gemäß Figur 1 mit einer Wabenanordnung in perspektivischer Darstellung ohne Außenhülle.

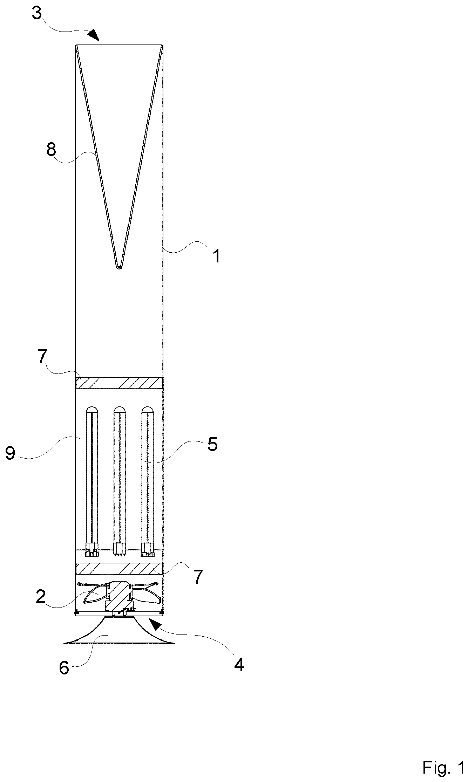

Figur 1 zeigt eine Querschnittsdarstellung einer Vorrichtung zur Reinigung von Raumluft. Der Aufbau sieht ein Luftleitelement 1 vor, welches ein Gehäuse der Vorrichtung darstellt. Es handelt sich um ein Rohrelement, in welchem eine Mehrzahl von UV-C-Leuchtmitteln 5 in Röhrenform aufgenommen sind. Diese werden elektrisch betrieben und weisen einen hier nicht gezeigten Neigungssensor auf, welcher im Falle einer Neigung des Luftleitelements 1 aus der Senkrechten heraus die UV-C-Leuchtmittel 5 von ihrer Spannungsversorgung trennen. Dies sorgt dafür, dass die in dem Luftleitelement 1 vorhandene UV-C-Strahlung nicht auf Personen gerichtet wird. Die Höhe des Luftleitelements 1 ist so gewählt, dass das Rohr zwei Meter über dem Boden endet, so dass ein Einblick in das aufrecht stehende Luftleitelement 1 verunmöglicht ist. Hierbei tritt die Luft aufgrund eines von einem Strömungsantrieb 2 erzeugten Unterdrucks über einen Lufteinlass 3 in das Luftleitelement 1 ein und fällt in einen kegelförmigen Staubfilter 8 hinein, welcher durch seine Kegelform eine besonders große Fläche abdecken kann. Sodann tritt die Luft durch eine Lamellenanordnung 7, welche einen Sichtschutz darstellt, welche einen Augenkontakt mit der schädlichen UV-C-Strahlung verhindert. Eine entsprechende Lamellenanordnung 7 findet sich jenseits der UV-C-Leuchtmittel im Bereich des Luftauslasses 4.

Um die UV-C-Leuchtmittel 5 herum ist ein zylindrischer UV-C-Reflektor 9 gebildet, welcher von den UV-C-Leuchtmitteln emittierte Strahlung von seinen Wänden in den Raum innerhalb des Luftleitelements 1 zurückwirft. Hierdurch erhöht sich die im Raum des Luftleitelements 1 vorhandene Energie, so dass eine größere Wahrscheinlichkeit besteht, dass Viren und Bakterien beim Durchlaufen des Luftleitelements 1 während der Passage des UV-C-Reflektors 9 zerstört werden.

Unterhalb der unteren Lamellenanordnung 7 ist der Strömungsantrieb 2 angeordnet, welcher die Luft aus dem Luftleitelement 1 axial ansaugt und axial nach unten hin abgibt. Hierbei lenkt der Strömungsantrieb 2 die Luft auf die Mantelfläche eines Standfußes 6, welcher kegelstumpfförmig gebildet ist und dabei eine konkave Mantelfläche aufweist. Aufgrund der konkaven Form wird die von dem Strömungsantrieb nach unten geleitete Luft zur Seite hin abgelenkt und kriecht über den den Standfuß umgebenden Boden, schiebt sich praktisch unter die anderen Luftschichten und vermeidet so effektiv das Auftreten von Verwirbelungen.

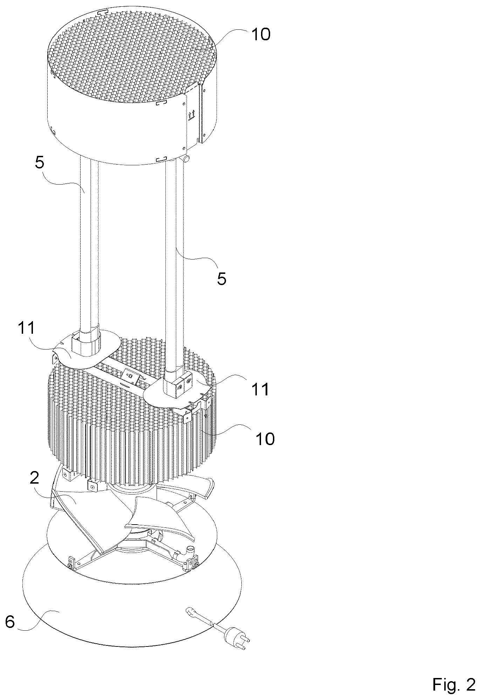

Figur 2 zeigt hingegen die Erfindung, bei der anstelle einer Lamellenanordnung eine Wabenanordnung 10 vorgesehen ist. In der Darstellung ist die Außenhülle und der eingangsseitige Staubfilter nicht dargestellt, sondern im Wesentlichen Wert auf die filterwirksamen Bestandteile gelegt worden. Ein Strömungsantrieb 2 saugt Luft von oberhalb ab und fördert diese abwärts in Richtung des Standfußes 6, über welchen die Luft seitlich umgelenkt und in den Raum verteilt wird, um diese möglichst unverwirbelt bodennah abzugeben. Hierdurch wird im Betrieb aufgrund der in Figur 2 ausgeblendeten Gehäuselemente die Luft durch eine über dem Strömungsantrieb 2 gelegene Wabenanordnung angesaugt. Diese gelangt über den oberseitigen Lufteinlass 3 in das Innere des Gehäuses und wird zunächst durch eine obere Wabenanordnung 10, an den UV-C-Leuchtmitteln vorbei, durch die untere Wabenanordnung 10 an dem Strömungsantrieb 2 vorbei geleitet. An den UV-C-Leuchtmitteln können sich zudem oben und/oder unten Abdeckbleche 11 befinden, welche einen direkten Lichtdurchfall durch die Wabenanordnung 10 verhindern, der mehr oder minder parallel zu den Wänden der einzelnen Waben innerhalb der Wabenanordnungen stattfinden müsste.

Vorstehend beschrieben ist somit eine Vorrichtung zur Reinigung von Raumluft, welche sich auch für die Reinigung der Raumluft von Viren eignet und die Erzeugung von zusätzlichen Verwirbelungen im Raum möglichst vermeidet.

### BEZUGSZEICHENLISTE

- 1: Luftleitelement
- 2: Strömungsantrieb
- 3: Lufteinlass
- 4: Luftauslass
- 5: UV-C-Leuchtmittel
- 6: Standfuß
- 7: Lamellenanordnung
- 8: Staubfilter
- 9: UV-C-Reflektor
- 10: Wabenanordnung
- 11: Abdeckblech

## Patentansprüche

1. Vorrichtung zur Reinigung von Raumluft, umfassend ein aufrecht stehendes, rohrförmiges und aus einem opaken Material hergestelltes Luftleitelement (1) mit einem Lufteinlass (3) und einem Luftauslass (4), sowie wenigstens einem zwischen diesen angeordneten, eine in dem Luftleitelement (1) abwärts gerichtete Strömung bewirkenden Strömungsantrieb (2) und einer dem Luftleitelement (1) inwendig zugeordneten Beleuchtungsvorrichtung, welche wenigstens ein UV-C-Leuchtmittel (5) umfasst,
**dadurch gekennzeichnet, dass** dem Luftleitelement (1) zwischen dem Lufteinlass (3) und dem wenigstens einen UV-C-Leuchtmittel (5) und zwischen dem Luftauslass (4) und dem wenigstens einen UV-C-Leuchtmittel (5) jeweils eine Wabenanordnung (10) mit Waben zugeordnet ist, welche lang im Vergleich zu ihrem Durchmesser sind, wobei dem Luftleitelement (1) ein die UV-C-Leuchtmittel (5) umgebender UV-C-Reflektor (9) zugeordnet ist, welcher aus einem zylindrisch geformten Aluminiumblech hergestellt ist, wobei dem Luftleitelement (1) unterhalb des Luftauslasses (4) ein Standfuß (6) zugeordnet ist, welcher kegelstumpfförmig ausgebildet ist und dabei eine konkave Mantelfläche aufweist, und wobei der Strömungsantrieb (2) unterhalb einer unteren Wabenanordnung (10) angeordnet ist, Luft aus dem Luftleitelement (1) axial ansaugt und axial nach unten hin abgibt, so dass der Strömungsantrieb (2) die Luft auf die Mantelfläche des Standfußes (6) lenkt.

2. Vorrichtung gemäß Anspruch 1, **dadurch gekennzeichnet, dass** die Wabenanordnung (10) aus einem Filtermaterial, insbesondere einem Vlies, einem Gewirk und/oder einem Textilmaterial, hergestellt ist.

3. Vorrichtung gemäß wenigstens einem der vorhergehenden Ansprüche, **dadurch gekennzeichnet, dass** es sich bei dem wenigstens einen UV-C-Leuchtmittel (5) um wenigstens eine Leuchtdiodenanordnung handelt, welche um eine Zentralachse herum in einem Abstrahlwinkel von zumindest näherungsweise 360° UV-C-Strahlung emittiert.

4. Vorrichtung gemäß wenigstens einem der Ansprüche 1 bis 3, **dadurch gekennzeichnet, dass** es sich bei dem wenigstens einen UV-C-Leuchtmittel (5) um wenigstens eine UV-C-Leuchtröhre handelt.

5. Vorrichtung gemäß wenigstens einem der vorhergehenden Ansprüche, **dadurch gekennzeichnet, dass** dem wenigstens einen UV-C-Leuchtmittel (5) ein Neigungsschalter zugeordnet ist, welcher eine elektrische Versorgungsleitung des wenigstens einen UV-C-Leuchtmittels (5) unterbricht, sobald eine Neigung, vorzugsweise eine Neigung von wenigstens 5°, gegenüber der Senkrechten festgestellt wird.

6. Vorrichtung gemäß wenigstens einem der vorhergehenden Ansprüche, **dadurch gekennzeichnet, dass** dem Luftauslass (4) Luftleitmittel zugeordnet sind, welche eine über den Luftauslass (4) austretende Luftströmung abwärts leiten.

7. Vorrichtung gemäß wenigstens einem der vorhergehenden Ansprüche, **dadurch gekennzeichnet, dass** dem Lufteinlass (3) ein Staubfilter (8) zugeordnet ist.

## Claims

1. A device for purifying room air, comprising an upright, tubular air guiding element (1) made of an opaque material having an air inlet (3) and an air outlet (4), as well as at least one flow drive (2) arranged therebetween and causing a downward flow in the air guiding element (1), and an illumination device internally associated with the air guiding element (1), which comprises at least one UV-C illuminant (5),
**characterized in that** between the air inlet (3) and the at least one UV-C illuminant (5) and between the air outlet (4) and the at least one UV-C illuminant (5), the air guiding element (1) is assigned a honeycomb arrangement (10) with honeycombs which are long in comparison with their diameter, the air guiding element (1) being assigned a UV-C reflector (9) which surrounds the UV-C illuminants (5) and is made from a cylindrically shaped aluminum sheet, wherein a base (6) is associated with the air guiding element (1) below the air outlet (4), which base is frustoconical in shape and has a concave outer surface, and wherein the flow drive (2) is arranged below a lower honeycomb arrangement (10), draws air axially from the air guiding element (1) and discharges it axially downwards, so that the flow drive (2) directs the air onto the outer surface of the base (6).

2. The device according to claim 1, **characterized in that** the honeycomb arrangement (10) is made of a filter material, in particular a nonwoven, a knitted fabric and/or a textile material.

3. The device according to at least one of the preceding claims, **characterized in that** the at least one UV-C illuminant (5) is at least one light-emitting diode arrangement which emits UV-C radiation around a central axis at a radiation angle of at least approximately 360°.

4. The device according to at least one of claims 1 to 3, **characterized in that** the at least one UV-C illuminant (5) is at least one UV-C fluorescent tube.

5. The device according to at least one of the preceding claims, **characterized in that** the at least one UV-C illuminant (5) is assigned a tilt switch which interrupts an electrical supply line of the at least one UV-C illuminant (5) as soon as an inclination, preferably an inclination of at least 5°, with respect to the vertical is detected.

6. The device according to at least one of the preceding claims, **characterized in that** air guide means are assigned to the air outlet (4), which guide an air flow exiting via the air outlet (4) downwards.

7. The device according to at least one of the preceding claims, **characterized in that** a dust filter (8) is assigned to the air inlet (3).

## Revendications

1. Un dispositif de purification de l'air ambiant, comprenant un élément de guidage d'air tubulaire vertical (1) en matériau opaque avec une entrée d'air (3) et une sortie d'air (4), ainsi qu'au moins un dispositif d'entraînement du flux (2) placé entre les deux et provoquant un flux descendant dans l'élément de guidage d'air (1), et un dispositif d'éclairage associé intérieurement à l'élément de guidage d'air (1), qui comprend au moins un illuminant UV-C (5),
**caractérisé en ce qu'**entre l'entrée d'air (3) et au moins un illuminant UV-C (5) et entre la sortie d'air (4) et au moins un illuminant UV-C (5), l'élément de guidage d'air (1) est doté d'un arrangement en nid d'abeilles (10) avec des nids d'abeilles longs par rapport à leur diamètre, l'élément de guidage d'air (1) étant doté d'un réflecteur UV-C (9) qui entoure les illuminants UV-C (5) et qui est fabriqué à partir d'une tôle d'aluminium de forme cylindrique, dans lequel une base (6) est associée à l'élément de guidage d'air (1) sous la sortie d'air (4), cette base étant de forme tronconique et présentant une surface extérieure concave, et dans lequel l'entraînement du flux (2) est disposé sous un arrangement inférieur en nid d'abeilles (10), aspire l'air axialement de l'élément de guidage d'air (1) et l'évacue axialement vers le bas, de sorte que l'entraînement du flux (2) dirige l'air sur la surface extérieure de la base (6).

2. Le dispositif selon la revendication 1, **caractérisé en ce que** l'arrangement en nid d'abeilles (10) est constitué d'un matériau filtrant, en particulier d'un non-tissé, d'un tissu tricoté et/ou d'un matériau textile.

3. Le dispositif selon au moins l'une des revendications précédentes, **caractérisé en ce que** l'au moins un illuminant UV-C (5) est au moins une diode électroluminescente qui émet un rayonnement UV-C autour d'un axe central à un angle de rayonnement d'au moins 360° environ.

4. Le dispositif selon au moins l'une des revendications 1 à 3, **caractérisé en ce que** l'au moins un illuminant UV-C (5) est au moins un tube fluorescent UV-C.

5. Le dispositif selon au moins une des revendications précédentes, **caractérisé en ce que** le au moins un illuminant UV-C (5) est équipé d'un interrupteur d'inclinaison qui interrompt une ligne d'alimentation électrique du au moins un illuminant UV-C (5) dès qu'une inclinaison, de préférence une inclinaison d'au moins 5°, par rapport à la verticale est détectée.

6. Le dispositif selon au moins l'une des revendications précédentes, **caractérisé en ce que** la sortie d'air (4) est équipée de moyens de guidage de l'air qui guident vers le bas le flux d'air sortant par la sortie d'air (4).

7. Le dispositif selon au moins l'une des revendications précédentes, **caractérisé en ce qu'**un filtre à poussière (8) est associé à l'entrée d'air (3).
